# EUROPÄISCHE PATENTSCHRIFT

(11) **EP 1 327 416 B1**
(45) Veröffentlichungstag und Bekanntmachung des Hinweises auf die Patenterteilung: **13.06.2007**
(21) Anmeldenummer: 03000822.1
(22) Anmeldetag: 15.01.2003
(51) Int. Cl.: A61B 8/00

(54) **Vorrichtung zur Ultraschallsonographie**
Device for the ultrasound sonography
Dispositif pour la sonographie à ultrason

(30) Priorität: 15.01.2002 DE 20200617 U
(43) Veröffentlichungstag der Anmeldung: 16.07.2003
(73) Patentinhaber: Compumedics Germany GmbH, 78224 Singen (DE)
(72) Erfinder: Sieb, Michael, 78354 Sipplingen (DE)
(74) Vertreter: Behrmann, Niels

(56) Entgegenhaltungen:
- DE-A1- 19 716 810
- US-A- 4 975 885
- US-A- 5 437 281
- US-A- 5 515 727
- US-B1- 6 248 071

## Beschreibung

Die vorliegende Erfindung betrifft eine Vorrichtung zur diagnostischen oder therapeutischen Ultraschallsonographie gemäß dem Hauptanspruch. Eine Vorrichtung nach dem Oberbegriff des Hauptanspruchs ist aus der US-B1-6248071 bekannt.

Im Stand der Technik ist durch Produkte der Anmelderin ein typischer Einsatz bei der intrakraniellen Gefäßbeobachtung und -diagnostik, etwa im Schädel eines Patienten, vorgesehen, allerdings mit analoger Mischertechnik.

Genauer gesagt wird eine geeignete Ultraschallsonde, typische Betriebsfrequenzen liegen im Bereich zwischen 2 und 2,5 MHz, am Kopf des Patienten befestigt, und eine Ultraschalleinheit erzeugt ein geeignetes Sendesignal für die Sonde. Das ebenfalls von der Sonde aufgenommene Empfangssignal wird dann einer analogen Mischereinheit zugeführt, mit dem Trägersignal gemischt, und das Dopplersignal als Mischprodukt wird dann mittels einer Tiefpaßfilterung abgetrennt und weiterer Verarbeitung zugeführt. Typischerweise wird dann aus diesem Dopplersignal eine visuelle Darstellung für ein in den Bildschirm einer Auswerteeinheit erzeugt, welche es der Bedienperson einer solchen Vorrichtung ermöglicht, Bewegungen (z.B. Blutfluß in den Gefäßen) zu erfassen und zu beurteilen. Auf einer derartigen Basisanwendung können dann zahlreiche weitere Diagnostiken und weitere Auswertungen aufgebaut sein, wie etwa das zusätzliche Erfassen von in einem Blutfluß auftretenden Embolien oder das gleichzeitige mehrkanalige Anzeigen einer Mehrzahl von Beobachtungstiefen.

Jede dieser bekannten Ultraschallvorrichtungen weist jedoch das prinzipbedingte Problem auf, dass die verwendeten Mischer zur Erzeugung des Dopplersignals als niederfrequentes Nutzsignal nicht über einen gesamten, gewünschten Amplituden- und Frequenzgang linear arbeiten; hinzu kommen gerade in Grenzbereichen störend auftretende Rauschkomponenten.

Zusätzlich ist es bei dieser bekannten, analogen Technik notwendig, jede vorgesehene Eindringtiefe (bestimmt durch die Laufzeit) separat aufzubauen, so dass der Hardware-Aufwand, insbesondere bei Vorrichtungen, die eine Mehrzahl von Tiefendarstellungen gleichzeitig vornehmen, beträchtlich ist, nicht zuletzt als für jede Tiefe (gate) zwei getrennte Kanäle (sinus und cosinus) für die Richtungserkennung der Blutflußgeschwindigkeit aufgebaut werden müssen.

Hinzu kommen herstellungstechnische Probleme, denn jede so hardwaretechnisch realisierte Eindringtiefe (gate) ist nach der Fertigung zeitaufwendig abzugleichen (sogenannter Spiegelabgleich), was wiederum zu beträchtlichem Zeitaufwand führt.

Aufgabe der vorliegenden Erfindung ist es daher, eine gattungsgemäße Vorrichtung zur diganostischen oder therapeutischen Ultraschallsonographie, insbesondere zur transkraniellen Gefäßdiagnostik, im Hinblick auf die Linearität des Amplituden- und Frequenzganges zu optimieren, Rauschen zu vermindern, und zusätzlich die Vorrichtung einfacher und effizienter sowie mit vermindertem Einstell- und Abgleichaufwand herstellen zu können.

Die Aufgabe wird durch die Vorrichtung mit den Merkmalen des Hauptanspruchs gelöst; vorteilhafte Weiterbildungen der Erfindung sind in den Unteransprüchen beschrieben.

In erfindungsgemäß vorteilhafter Weise sorgt der Einsatz eines empfangsseitig mittels eines AD-Wandlers unmittelbar an die Sonde angekoppelten, digitalen Mischers dafür, dass zunächst die Linearitätsprobleme analoger Mischer im Hinblick auf Frequenzgang und Amplitude überwunden werden; digital sind die Betriebsgrenzen sowie das Rauschen lediglich durch die Auflösung des Digitalsignals (bzw. dessen Signalfrequenz/bit-Rate) bestimmt und hängen mithin von der Leitungsfähigkeit und der Rechenkapazität der nachgeschalteten digitalen Signalverarbeitungselektronik ab. In ansonsten bekannter Weise besteht der digitale Mischer aus einem binären Multiplizierer, der innerhalb seiner Auflösung (bit-Breite) völlig linear ist, und dessen Bandbreite nur von der Abtastrate der zu mischenden Signale abhängt. Damit wird das System konfigurierbar, und während in besonders bevorzugter Weise typische Sondenfrequenzen zwischen 2 und 4 MHz digital unterstützt werden können, ist prinzipiell jede Sondenfrequenz möglich, da sowohl die Sendefrequenz als auch die Mischerfrequenz durch geeignete digitale Ansteuerung einstellbar sind; entsprechend lassen sich die eingesetzten digitalen Filter auf die Arbeitsfrequenz konfigurieren.

Die Mischfrequenz (Sinus-/Cosinus-Anteil) wird durch einen digitalen Frequenzgenerator als Impulsgenerator erzeugt, wobei sich hierfür ein Speicher mit Sinus-/Cosinus-Tabellenwerten als besonders bevorzugt herausgestellt hat, welcher dann mittels einer Zählereinheit ausgelesen wird. Sendeseitig wird bevorzugt dieses Digitalsignal des Impulsgenerators zur PRF-Erzeugung (PRF = Pulswiederholfrequenz) für das Sendesignal digital geformt und dann (über einen geeigneten Leistungsverstärker) sendeseitig an die Sonde angelegt.

Beim Einsatz von Ultraschallsonden im typischen Bereich zwischen 2 und 4 (bzw. 8) MHz und geeignet eingestellter Abtastrate liegt ein typisches, empfangsseitig vom Analog-Digital-Wandler erzeugtes Eingangssignal in der Größenordnung von 12 MHz, wobei, nach der digitalen Mischung, mit Hilfe von digitalen Tiefpaßfiltern, diese Datenrate mehrstufig und schrittweise auf eine Größenordnung zwischen 1 und 2 MHz reduziert wird (eine Frequenz von 1,5 MHz entspricht einer Auflösung von ca. 1 mm, bezogen auf die Schallgeschwindigkeit in Wasser).

Besonders bevorzugt ist es zudem im Rahmen der Erfindung, mit Hilfe der weiterbildungsgemäß vorgesehenen Signalaufbereitungseinheit bereits die gewünschten Tiefensignale aus dem Datenstrom abzutrennen, damit die nachfolgende Verarbeitung im Rechner vereinfacht (beschleunigt bzw. entlastet) werden kann; alternativ oder ergänzend ist es möglich, dieses abgetrennte Signal vorteilhaft zu verwenden, um eine sogenannte M-Mode-Darstellung (visuelle Darstellung des Amplitudenbetrages über der Tiefe) darzustellen. Hier zeigt sich jeweils der Vorteil der Erfindung durch erhöhte Genauigkeit bzw. Vermeidung von Nichtlinearitäten (etwa im Vergleich zu einem analogen Mischer).

Im Ergebnis wird durch die vorliegende Erfindung vorteilhaft erreicht, dass optimierte bzw. voreinstellbare Rauscheigenschaften mit perfekter Linearität kombiniert werden, und, da durch rechnergesteuert einstellbare Mischer- und Sendefrequenz sowie Konfigurierung der Filter praktisch eine beliebige Einstellbarkeit auf gewünschte Tiefen möglich ist, wird durch die erfindungsgemäße Vorrichtung eine ungeahnte Flexibilität im Einsatz, verbunden mit geringstmöglichem Einstell- und manuellen Konfigurationsaufwand erreicht. Hinzu kommt der Vorteil, dass die Anzahl der darzustellenden Tiefen (letztendlich ja durchgehend im digitalen Dopplersignal enthalten) nur durch die vorhandene Rechnerleistung begrenzt ist, so dass auch hier mit geringstem Hardware- und Herstellungsaufwand beachtliche Vorteile realisiert werden können.

Weitere Vorteile, Merkmale und Einzelheiten der Erfindung ergeben sich aus der nachfolgenden Beschreibung bevorzugter Ausführungsbeispiele sowie anhand der einzigen Zeichnung; diese zeigt in

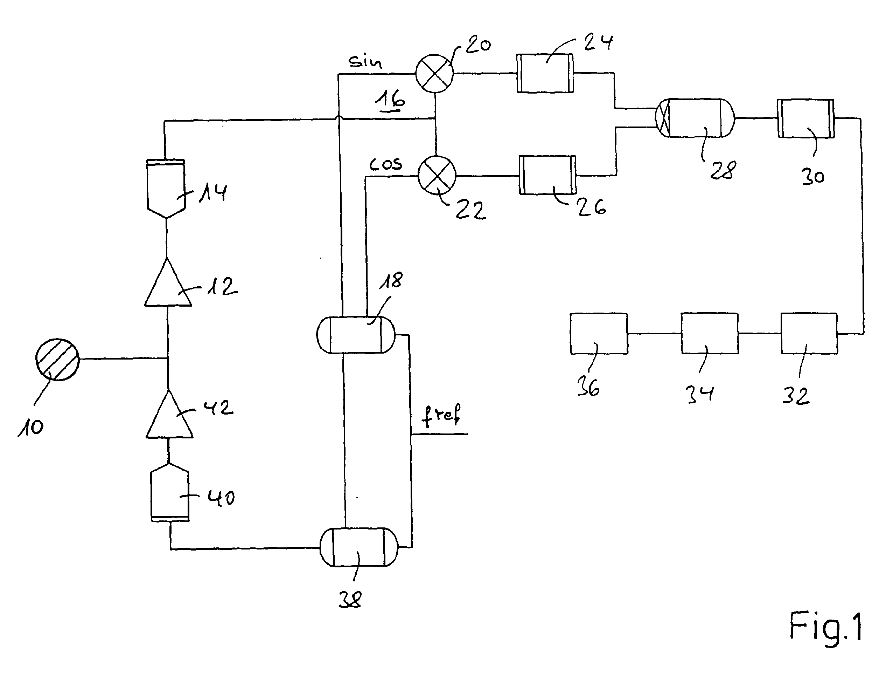

Fig. 1 ein schematisches Blockschaltbild der erfindungsgemäßen Vorrichtung zur analytischen oder therapeutischen Ultraschallsonographie gemäß einer bevorzugten Ausführungsform.

Das Ausgangssignal einer in ansonsten bekannter Weise am Kopf eines Patienten befestigten Ultraschallsonde 10 (typische Betriebsfrequenz 2 oder 2,5 MHz) wird über einen Vorverstärker 12 auf der Empfangsseite einer Analog-Digital-Wandlereinheit 14 zugeführt, welche das verstärkte Empfangssignal schnell und mit hoher Auflösung (typische Signalfrequenz am Ausgang des AD-Wandlers 14: 12 MHz) digitalisiert und einem digitalen Mischer 16 zuführt, welcher, getrennt für einen Sinusanteil und einen Cosinusanteil, das digitale Eingangssignal mit der Sinuskomponente bzw. der Cosinuskomponente eines von einem Signalerzeuger 18 auf der der Sondenfrequenz entsprechenden Trägerfrequenz erzeugten Mischersignals mischt.

Genauer gesagt wird in dem digitalen Mischer 16 mit Hilfe von dem Sinus- bzw. dem Cosinuszweig jeweils zugeordneten digitalen Multiplizierern 20, 22 das digitale Multiplikationsprodukt des digitalisierten Empfangssignals (aus der Einheit 14) sowie der Sinus- bzw. Cosinuskomponente des von der Einheit 18 erzeugten Trägerfrequenzsignals gebildet, so dass, in der bekannten Weise, am Ausgangs der Multiplizierer 20, 22 die Doppler-Mischfrequenzen gebildet werden, wobei dann jeweils nachgeschaltete digitale Filtereinheiten 24, 26, als Tiefpaß wirkend, das gewünschte, dem Dopplershift entsprechende Signal (entsprechend der Differenz von Eingangssignal und Mischerfrequenz) erzeugen. Konkret sind diese Filter als die Datenrate reduzierende FIR-Filter realisiert und bewirken eine Reduzierung der Datenrate von 1:2, so dass einer nachgeschalteten digitalen Multiplexereinheit 28 zwei 6 MHz-Datenströme zufließen.

Die Multiplexereinheit 28 erzeugt aus diesen Signalen ein 6 MHz Ausgangssignal, welches dann in einer nachgeschalteten digitalen Tiefpaßfilterstufe 30 nochmals in der Datenrate um 1:4 reduziert wird, so dass einer nachgeschalteten Sample-Speichereinheit 32 als Signalaufbereitungseinheit im Sinne der Erfindung Digitalsignale einer Datenrate von ca. 1,5 MHz anliegen. Mit einer Auflösung von ca. einem Millimeter (bezogen auf die Schallgeschwindigkeit in Wasser) liegen typische, über einen verbundenen Hostrechner (nicht gezeigt) einstellbare Tiefen der Ultraschallmessung zwischen 20 und 150 mm, wobei, wie beschrieben, die eingesetzte digitale Mischertechnologie für vollständige Linearität entlang dieses Bereiches sorgt. Besonders bevorzugt ist es, geeignet vorgewählte bzw. voreingestellte Tiefen durch entsprechende Ansteuerung dieser Sample-Speichereinheit als Werte unmittelbar auszulesen und einer gesonderten Verarbeitung, etwa zur M-Mode-Darstellung, zuzuleiten.

Der Gesamtdatenstrom tritt zudem in einen nachgeschalteten FIFO-Pufferspeicher 34 und von diesem in eine Rechnerschnittstelle 36 ein, welche, z.B. als PCI-Schnittstelle, die Verbindung zu handelsüblicher PC-Rechnerinfrastruktur herstellt und die vollständige signaltechnische Aufbereitung und Darstellung des Dopplersignals in ansonsten bekannter Weise ermöglicht.

Senderseitig sorgt der digitale Signalerzeuger 18 zudem dafür, das PRF-Signal für eine nachgeschaltete Signalformereinheit 38 zur Ansteuerung bereitzustellen, wobei das Ausgangssignal dieser Einheit (sogenanntes Burstsignal) dann über eine Digital-Analog-Wandlereinheit 40 und einen nachgeschalteten Leistungsverstärker 42 an die Ultraschallsonde 10 angelegt wird.

Genauer gesagt ist die mit der Einheit 38 realisierte digitale Signalformung programmierbar und wird, ebenso wie die Signalvorverarbeitung auf Empfangsseite wie obenstehend beschrieben, durch die verbundene Hostrechnereinheit geeignet programmtechnisch gesteuert. Die Einheiten 18 und 38 empfangen zudem eine geeignet gewählte Referenzfrequenz fref (z.B. 48 MHz).

Konkret wird in der praktischen Realisierung die digitale Mischerelektronik (d.h. Einheiten 20, 22) samt Impulsgenerator 18 und digitalem Signalformer 38 als Digitalbaustein integriert und liegt typischerweise als geeignet konfigurierter Chip vor, so dass, zusammen mit geeignet durch den Rechner implementierten Filtereinheiten bzw. der Multiplexer- und der Sample-Speichereinheit, die erfindungsgemäße Vorrichtung herstellungstechnisch einfach realisierbar ist und praktisch keinen manuellen Kalibrierungs- bzw. Einstellaufwand erfordert.

Im Ergebnis läßt sich durch die vorliegende Erfindung, verdeutlicht am Ausführungsbeispiel der Fig. 1, in überraschend einfacher und flexibler Weise eine Dopplerdemodulation realisieren, welche, in Abkehr von der bislang ausschließlich im Diagnostikbereich benutzten analogen Technologie in bisher ungeahnter Weise Linearität entlang des gesamten nutzbaren Amplituden- und Frequenzbereiches mit lediglich von der digitalen Auflösung bestimmter Rauscharmut kombiniert.

## Patentansprüche

1. Vorrichtung zur diagnostischen oder therapeutischen Ultraschallsonographie mit
einer zum Verbinden mit einer Ultraschallsonde (10) zum Erzeugen von Ultraschallsignalen sowie zum Empfangen reflektierter Ultraschallsignale ausgebildeten Ultraschalleinheit,
die einen Impulsgenerator (18) für die sendeseitig erzeugten periodischen Ultraschallsignale sowie empfangsseitig eine Mischereinheit (16) und eine Filtereinheit (24,26,30) aufweist und zum Verbinden mit einer Signalauswerteeinheit (PC) zum Aufbereiten und visuellen Darstellen von aus den reflektierten Ultraschallsignalen erzeugten Daten eingerichtet ist, wobei der Ultraschallsonde empfangsseitig eine Analog-Digital-Wandlereinheit (14) nachgeschaltet ist, deren digitales Ausgangssignal in der als digitaler Mischer ausgebildeten Mischereinheit (16,20,22) mit einem Mischersignal des Impulsgenerators (18) digital gemischt werden kann,
**dadurch gekennzeichnet, dass**
die Ultraschalleinheit empfangsseitig so ausgebildet ist, dass ein einem Sinus-Mischersignal entsprechender Ausgangszweig und ein einem Cosinus-Mischersignal entsprechender Ausgangszweig mit einer digitalen Multiplexereinheit (28) zum Erzeugen eines gemultiplexten digitalen Dopplersignals verbunden ist.

2. Vorrichtung nach Anspruch 1, **dadurch gekennzeichnet, dass** dem digitalen Mischer eine digitale, als Tiefpaßfilter wirkende Filtereinheit (24,26), insbesondere FIR-Filter, nachgeschaltet ist.

3. Vorrichtung nach Anspruch 1 oder 2, **dadurch gekennzeichnet, dass** zwischen dem digitalen Mischer (16) und der digitalen Multiplexereinheit (28)in jedem Ausgangszweig ein digitales Tiefpaßfilter (24, 26)vorgesehen ist.

4. Vorrichtung nach einem der Ansprüche 1 bis 3, **dadurch gekennzeichnet, dass** der Multiplexereinheit eine als Tiefpaßfilter wirkende Filtereinheit (30), insbesondere FIR-Filter, nachgeschaltet ist.

5. Vorrichtung nach einem der Ansprüche 1 bis 4, **dadurch gekennzeichnet, dass** der Multiplexereinheit eine Signalaufbereitungseinheit (32) nachgeschaltet ist, die der Signalauswerteeinheit vorgeschaltet und so ausgebildet ist, dass sie aus einem Datenstrom der Multiplexereinheit eine vorbestimmte, der gewünschten Beobachtungstiefe entsprechende Datenkomponente abtrennt und der Signalauswerteeinheit zuleitet.

6. Vorrichtung nach Anspruch 5, **dadurch gekennzeichnet, dass** die Signalauswerteeinheit und/oder die Signalaufbereitungseinheit so ausgebildet sind, dass sie aus den abgetrennten Datenkomponenten eine einem M-Mode entsprechende Bilddarstellung auf einem Bildschirm oder dergleichen Bildausgabeeinheit der Signalauswerteeinheit erzeugen.

7. Vorrichtung nach einem der Ansprüche 1 bis 6, **dadurch gekennzeichnet, dass** der Impulsgenerator (18) als digitaler Frequenzgenerator ausgebildet ist, der, insbesondere mittels einer zugeordneten digitalen Tabelleneinheit, ein Mischersignal mit Sinus- und Cosinusanteil für den digiten Mischer (16,20,22) bereitstellt und sendeseitig einen digitalen Impulsformer (38) zur Erzeugung des Sendesignals ansteuert.

8. Vorrichtung nach einem der Ansprüche 1 bis 7, **dadurch gekennzeichnet, dass** der digitale Mischer (16), der Impulsgenerator (18) sowie ein sendeseitig vorgesehener, dem Impulsgenerator nachgeschalteter Impulsformer (38) als integriertes Halbleiterbauelement realisiert und bevorzugt programmtechnisch einstell- oder beeinflußbar sind.

9. Vorrichtung nach einem der Ansprüche 1 bis 8, **gekennzeichnet durch** eine Betriebsfrequenz der Ultraschallsonde im Bereich zwischen 1,5 und 12 MHz, insbesondere zwischen 2 und 8 MHz, wobei ein dem digitalen Mischer empfangsseitig zugeleiteter digitaler Datenstrom aus der Analog-Digital-Wandlereinheit (14) eine Frequenz im Bereich zwischen 10 und 15 MHz aufweist.

10. Vorrichtung nach Anspruch 9, **dadurch gekennzeichnet, dass** eine der Signalauswerteeinheit zugeleitete Signalfrequenz des tiefpaßgefilterten und gemultiplexten Mischerausgangssignals als Dopplersignal im Bereich zwischen 1 und 2 MHz liegt.

11. Verwendung der Vorrichtung nach einem der Ansprüche 1 bis 10 als Vorrichtung zur intrakraniellen Gefäßbeobachtung und -diagnostik.

## Claims

1. Device for diagnostic or therapeutic ultrasonography, comprising an ultrasonic unit designed to be connected to an ultrasonic probe (10) for generating ultrasonic signals and for receiving reflected ultrasonic signals, provided with a pulse generator (18) for the periodic ultrasonic signals generated at the transmitting end and, at the receiving end, with a mixer unit (16) and a filter unit (24, 26, 30), and designed to be connected to a signal evaluation unit (PC) for conditioning and visually displaying data generated from the reflected ultrasonic signals, an analog-digital converter unit (14) the digital output signal of which can be mixed digitally with a mixer signal of the pulse generator (18) in the mixer unit (16, 20, 22) designed as a digital mixer being arranged downstream of the ultrasonic probe at the receiving end, **characterised in that** the ultrasonic unit is designed in such a manner at the receiving end that an output branch corresponding to a sine mixer signal and an output branch corresponding to a cosine mixer signal are connected to a digital multiplexer unit (28) for generating a multiplexed digital Doppler signal.

2. Device according to claim 1, **characterised in that** a digital filter unit (24, 26) acting as a low-pass filter, in particular an FIR filter, is arranged downstream of the digital mixer.

3. Device according to claim 1 or claim 2, **characterised in that** a digital low-pass filter (24, 26) is provided in each output branch between the digital mixer (16) and the digital multiplexer unit (28).

4. Device according to one of claims 1 to 3, **characterised in that** a filter unit (30) acting as a low-pass filter, in particular an FIR filter, is arranged downstream of the multiplexer unit.

5. Device according to one of claims 1 to 4, **characterised in that** a signal-conditioning unit (32) arranged upstream of the signal evaluation unit is arranged downstream of the multiplexer unit and is designed in such a manner that it separates a predetermined data component corresponding to the desired viewing depth from a data stream of the multiplexer unit and feeds it to the signal evaluation unit.

6. Device according to claim 5, **characterised in that** the signal evaluation unit and/or the signal-conditioning unit are designed in such a manner that they generate an image display corresponding to an M-mode on a screen or similar image output unit of the signal evaluation unit from the separated data components.

7. Device according to one of claims 1 to 6, **characterised in that** the pulse generator (18) is designed as a digital frequency generator which, in particular by means of an associated digital table unit, supplies a mixer signal with a sine component and a cosine component for the digital mixer (16, 20, 22) and activates a digital pulse shaper (38) at the transmitting end for generating the transmission signal.

8. Device according to one of claims 1 to 7, **characterised in that** the digital mixer (16), the pulse generator (18) and a pulse shaper (38) provided downstream of the pulse generator at the transmitting end are produced as an integrated semiconductor component and can preferably be adjusted or influenced by programming.

9. Device according to one of claims 1 to 8, **characterised by** an operating frequency for the ultrasonic probe in the range of between 1.5 and 12 MHz, in particular between 2 and 8 MHz, a digital data stream fed from the analog-digital converter unit (14) to the digital mixer at the receiving end having a frequency in the range of between 10 and 15 MHz.

10. Device according to claim 9, **characterised in that** a signal frequency of the low-pass multiplexed mixer output signal in the form of a Doppler signal fed to the signal evaluation unit is in the range of between 1 and 2 MHz.

11. Use of the device according to one of claims 1 to 10 as a device for intracranial vascular monitoring and diagnostics.

## Revendications

1. Dispositif pour la sonographie à ultrasons, diagnostique ou thérapeutique, comprenant:
- une unité à ultrasons montée de façon à se raccorder à une sonde à ultrasons (10) pour produire des signaux ultrasons, ainsi que pour recevoir des signaux ultrasons réfléchis,
- qui présente un générateur d'impulsions (18) pour les signaux ultrasons périodiques produits côté émetteur, ainsi qu'une unité de mélangeur (16) et une unité de filtrage (24,26,30) côté récepteur, et qui est aménagée de façon à se raccorder à une unité d'évaluation du signal (PC) pour traiter et représenter visuellement des données produites à partir des signaux ultrasons réfléchis, dans lequel est connectée, côté récepteur, en aval de la sonde à ultrasons, une unité de conversion analogique-numérique (14) dont le signal de sortie numérique peut être mélangé numériquement avec un signal de mélangeur du générateur d'impulsions (18) dans l'unité de mélangeur (16,20,22) réalisée sous forme de mélangeur numérique,
**caractérisé en ce que**
- l'unité à ultrasons est montée côté récepteur, de telle sorte qu'une branche de sortie correspondant à un signal de mélangeur sinus et une branche de sortie correspondant à un signal de mélangeur cosinus, sont raccordées à une unité de multiplexage numérique (28) pour produire un signal Doppler numérique multiplexé.

2. Dispositif selon la revendication 1, **caractérisé en ce qu'**une unité de filtrage numérique (24, 26) agissant comme filtre passe-bas, en particulier un filtre RIF, est connectée en aval du mélangeur numérique.

3. Dispositif selon la revendication 1 ou 2, **caractérisé en ce qu'**un filtre passe-bas numérique (24, 26) est prévu dans chaque branche de sortie entre le mélangeur numérique (16) et l'unité de multiplexage numérique (28).

4. Dispositif selon l'une des revendications 1 à 3, **caractérisé en ce qu'**une unité de filtrage (30) agissant comme filtre passe-bas, en particulier un filtre RIF, est connectée en aval de l'unité de multiplexage.

5. Dispositif selon l'une des revendications 1 à 4, **caractérisé en ce que**, en aval de l'unité de multiplexage, est connectée une unité de traitement du signal (32) qui est connectée en amont de l'unité d'évaluation du signal, et conçue de telle sorte qu'elle sépare, à partir d'un flux de données de l'unité de multiplexage, une composante de données prédéterminée correspondant à la profondeur d'observation souhaitée, et la transmet à l'unité d'évaluation du signal.

6. Dispositif selon la revendication 5, **caractérisé en ce que** l'unité d'évaluation du signal et/ou l'unité de traitement du signal sont conçues de telle sorte qu'elles produisent, à partir des composantes de données séparées, une représentation visuelle correspondant à un mode M, sur un écran ou une unité d'affichage d'images similaire, de l'unité d'évaluation du signal.

7. Dispositif selon l'une des revendications 1 à 6, **caractérisé en ce que** le générateur d'impulsions (18) est réalisé en tant que générateur de fréquence numérique qui, en particulier au moyen d'une unité de tableau numérique dédiée, met à disposition un signal de mélangeur avec une composante sinus et une composante cosinus pour le mélangeur numérique (16,20,22), et commande un formateur d'impulsions numérique (38) côté émetteur pour produire le signal d'émission.

8. Dispositif selon l'une des revendications 1 à 7, **caractérisé en ce que** le mélangeur numérique (16), le générateur d'impulsions (18) ainsi qu'un formateur d'impulsions (38) prévu côté émetteur, connecté en aval du générateur d'impulsions, sont réalisés sous forme d'un composant semi-conducteur intégré et, de préférence, susceptibles d'être réglés ou influencés par une technique de programmation.

9. Dispositif selon l'une des revendications 1 à 8, **caractérisé par** une fréquence de fonctionnement de la sonde à ultrasons comprise entre 1,5 et 12 MHz, en particulier entre 2 et 8 MHz, dans lequel un flux de données numérique transmis côté récepteur au mélangeur numérique, à partir de l'unité de conversion analogique-numérique (14), présente une fréquence comprise entre 10 et 15 MHz.

10. Dispositif selon la revendication 9, **caractérisé en ce qu'**une fréquence de signal, transmise à l'unité d'évaluation du signal, du signal de sortie du mélangeur passé par le filtre passe-bas et multiplexé, se trouve sous forme de signal Doppler entre 1 et 2 MHz.

11. Utilisation du dispositif selon l'une des revendications 1 à 10, en tant que dispositif pour l'observation et le diagnostic des vaisseaux intracrâniens.
